# EUROPEAN PATENT APPLICATION

(11) **EP 3 653 124 A1**
(43) Date of publication of application: **20.05.2020**
(21) Application number: 18206485.7
(22) Date of filing: 15.11.2018
(51) Int. Cl.: A61B 6/00, A61B 6/08, A61B 5/00, A61B 5/107

(54) **SYSTEM FOR ADJUSTING A RELATIVE POSITION OF AN INTERIOR BODY PORTION RELATIVE TO AN X-RAY SENSITIVE SURFACE**

(71) Applicant: Koninklijke Philips N.V., 5656 AG Eindhoven (NL)
(72) Inventor: MAACK, Hanns-Ingo, 5656 AE Eindhoven (NL)
(74) Representative: de Haan, Poul Erik

(57) **Abstract**

The present disclosure relates to a system for adjusting, for each of a plurality of subjects under inspection, a relative position and/or a relative orientation between an interior body portion of the respective subject and an X-ray sensitive surface of an X-ray sensitive device. The system comprises a data processing system which is configured to read, for each of the subjects, position data which are indicative of one or more parameters of a position of the respective subject. The data processing system is further configured to determine one or more position parameters of an interior body portion of the respective subject. The data processing system is further configured to determine the position parameters of the interior body portions of the subjects further depending on model data which are indicative of one or more parameters of an interior position of an interior body portion of a model subject within a body of the model subject.

## Description

### FIELD OF THE INVENTION

The present invention relates to a system for automatically and/or interactively adjusting a relative position and/or a relative orientation between an interior body portion of a subject under inspection and an X-ray sensitive surface of an X-ray sensitive device. Specifically, the present invention relates to an X-ray detection system for use in a system for X-ray radiography. The X-ray detection system has a radiation dose sensor or for controlling an exposure of an X-ray detector detector and the X-ray detection system is configured for automatic and/or interactive adjustment of a position and/or extent of a radiation-sensitive surface of the radiation dose sensor.

### BACKGROUND OF THE INVENTION

Known in the prior art are X-ray imaging systems which are provided with an automatic exposure control which is configured to deliver consistent and reproducible exposures. Such automatic exposure control systems are typically based on X-ray sensitive detectors, such as ionization chambers, solid-state detectors, and a combination of fluorescent screens with photomultiplier tubes or photodiodes. During imaging, the exposure control system generates a signal, which is indicative of the radiation dose. Depending on the signal, the exposure control system controls the X-ray source so that on the one hand, the radiation dose is sufficient for obtaining a radiograph, which allows a reliable diagnosis and on the other hand, the radiation dose does not lead to overexposure of the patient.

However, it has been shown that correct determination of the radiation dose critically depends on the position of the interior body portion under inspection relative to the radiation dose sensor. The radiologist or radiographer therefore has to rely on his experience to properly positon the subject in front of the detector. Further, it has been shown that a fixed position and extent of the radiation dose sensor makes the automatic exposure control not suitable for paediatric imaging. Notably, the constant growth that occurs during childhood results in changing body proportions so that it is difficult to use these fixed radiation dose sensors for all age ranges.

Therefore, a need exists for an improved system, method, computer program element and computer-readable medium which allow improved X-ray imaging.

### SUMMARY OF THE INVENTION

Embodiments of the present invention pertain to a system for automatically or interactively adjusting, for each of a plurality of subjects, a relative position and/or a relative orientation between an interior body portion of the respective subject under inspection and an X-ray sensitive surface of an X-ray sensitive device. The system comprises a data processing system which is configured to read and/or to determine, for each of the subjects, position and/or orientation data which are indicative of one or more parameters of a position and/or an orientation of the respective subject. The data processing system is further configured to determine, for each of the subjects, one or more position and/or orientation parameters of an interior body portion of the respective subject. The data processing system is further configured to determine, for each of the subjects, the one or more position and/or orientation parameters of the interior body portion of the respective subject further depending on same model data. The model data are indicative of one or more parameters of an interior position and/or an interior orientation of an interior body portion of a model subject within a body of the model subject.

The system may be configured for use with in an X-ray imaging system, which is configured to generate X-ray radiographs of a body portion of a human subject. The X-ray sensitive device may be at least a portion of an X-ray radiation dose sensor. The X-ray radiation dose sensor may be configured to monitor a dose of X-ray radiation, which is absorbed at the X-ray sensitive surface. Additionally or alternatively, the X-ray sensitive device may be at least a portion of an X-ray sensitive image detector.

The position and/or orientation data may be indicative of one or more parameters of a position and/or orientation of the subject relative to the X-ray sensitive surface and/or relative to an X-ray sensitive surface of an X-ray image detector for detecting an X-ray image of the body portion.

Examples for parameters of a position are but are not limited to one or more coordinate values of one or more coordinate axes. Examples for parameters of an orientation are but are not limited to rotation angle values for one or more rotation axes.

A sampling rate of the radiation dose sensor for measuring the radiation dose may be equal to or greater than 1 kHz, equal to or greater than 2 kHz, equal to or greater than 5 kHz, or equal to or greater than 10 kHz.

The data processing system may include a computer system having a processor and a memory for storing instructions processable by the processor. The processor may execute an operating system. The processor may perform operations to perform the method steps and operations discussed within the present disclosure. The data processing system may further include a user interface, in particular a graphical user interface. The user interface may be configured to allow a user to receive data from the data processing system and/or may allow the user to provide data to the data processing system. The user input may be received via input devices of the data processing system, such as a computer mouse and/or a keyboard. The graphical user interface may include a display device.

The data processing system may be configured to receive user input, which represents the position and/or orientation data. Additionally or alternatively, the data processing system may be in signal communication with a position and/or orientation data acquisition device of the system, which is configured to acquire the position and/orientation data from the subject.

The interior body portion may be located within an interior of the body. The model data may represent a model of the interior position and/or the interior orientation of the interior body portion of the model subject within the body of the model subject. The body portion of the model and the body portions of the subjects may correspond (i.e. being of a same type). A type of interior body portions may be defined as an anatomically and/or a functionally defined portion of the body. An anatomically defined portion of the body may be a bone structure and/or a tissue structure of the body. A functionally defined portion of the body may be a portion of the body, which performs an anatomical function. Examples for types of interior body portions are but are not limited to at least a portion of: a lung, a knee, an extremity, such as a knee joint.

The model data are used for each of the plurality of subjects. The model subject may represent a model, which is applied for determining one or more position and/or orientation parameter of the interior body portion of a plurality of different subjects. The model data may be generated using data acquired from one or more subjects other than the subjects under inspection. By way of example, the model data may be acquired using data acquired from more than 5, or more than 10, or more than 20, or more than 50 subjects.

By way of example, the model data may include one or more coordinate values of a shape of the interior body portion and/or one or more coordinates of an external body shape of the model subject.

The determining of the one or more position and/or orientation parameters of the interior body portion of the respective subject may include performing a statistical fit between at least a portion of the model data and the position and/or orientation data of the position and/or orientation of the subject.

According to an embodiment, the data processing system is configured to read and/or to generate, for each of the subjects, selection data for selecting the model data from a plurality of model data sets. Each of the model data sets may be associated with one or more model subjects. The data processing system may be further configured to select, for each of the subjects, the model data depending on the selection data.

According to an embodiment, the selection data are indicative of, or are determined by the data processing system depending on, one or parameters of at least one attribute of the subject under inspection. Examples for attributes are but are not limited to: age and physical attributes. Examples of physical attributes are but are not limited to: gender, weight, body mass index (BMI) and a body dimension. Examples for body dimensions are but are not limited to: the body height of the subject, a length an extremity, a thickness of an irradiated portion of the subject (such as the thorax of the subject), which is irradiated by the X-rays, wherein the thickness is measured in a direction of at least a portion of the X-rays. The direction may be measured in a direction perpendicular to an image plane of the detector. A further example is a thickness of the irradiated portion of the subject (such as the thorax) in a direction perpendicular to the direction of the portion of the X-rays. The direction perpendicular to the direction of the portion of the X-rays may be measured perpendicular or along a longitudinal axis of the body.

According to a further embodiment, the X-ray sensitive device is a radiation dose sensor. The radiation dose sensor may be configured to generate radiation dose measurement signals which are indicative of a radiation dose absorbed by the X-ray sensitive surface. The system may further include a controller which is configured to receive the radiation dose measurement signals and to generate a control signal for controlling an exposure of an X-ray source depending on the radiation dose measurement signals.

The radiation dose sensor may be configured to generate a radiation dose measurement signal for each of a plurality of sampling points in time. The sampling points may be during an exposure time interval during which the X-ray sensitive surface is continuously exposed to radiation generated by the X-ray source. The radiation dose signal may be indicative of the radiation dose absorbed by the X-ray sensitive surface of the radiation dose sensor since the start of the exposure time interval.

The control signal for controlling the X-ray source may be a signal to deactivate generation of X-rays which are generated by the X-ray source. The control signal may be configured to terminate the exposure time interval. By way of example, the signal may be configured to stop an impingement of an electron beam, which is generated by the X-ray source on a target of the X-ray source.

According to a further embodiment, the system for adjusting the relative position and/or orientation between the interior body portion and the X-ray sensitive surface may include the X-ray source, wherein the X-ray source is configured to receive the control signals generated by the controller.

The data processing system may be configured to receive at least a portion of the selection data from the user via a user interface. The model subjects may relate to different attributes, in particular different physical attributes.

Examples for attributes are but are not limited to: age and physical attributes. Examples for physical attributes are but are not limited to: gender, weight, body mass index (BMI) and one or more body dimensions. Examples for body dimensions are but are not limited to: the body height of the subject and a length of one or more extremities.

According to a further embodiment, the data processing system is configured to read and/or to generate, for each of the subjects, two-dimensional and/or three-dimensional image data which show at least a portion of a body of the respective subject. The data processing system may be further configured to determine, for each of the subjects, the position and/or orientation data depending on the image data acquired for the respective subject. Additionally or alternatively, the data processing system may be configured to determine, for each of the subjects, the selection data depending on the two-dimensional and/or three-dimensional image data acquired for the respective subject.

The two-dimensional and/or the three-dimensional image data may be acquired using an image acquisition system, which is configured to acquire at least a portion of the two-dimensional and/or at least a portion of the three-dimensional image data. The image acquisition system may include an image sensor. The image sensor may be sensitive to light having wavelengths in the visible spectral range. The visible spectral range may be defined as a range from 380 to 750 nanometers. Additionally or alternatively, the image acquisition system may include an imaging optical system which is configured to image the subject onto the image sensor. The image optical system may include one or more lenses. By way of example, the image acquisition system may be configured to perform photogrammetry. Additionally or alternatively, three-dimensional image data may be acquired using at least two image acquisition systems which are configured to acquire image data from the subject from at least two different imaging directions. Each of the image acquisition systems may include an image optical system having an optical axis and/or one or more lenses. Each of the imaging direction may correspond to an optical axis of one of a plurality of image optical systems.

Additionally or alternatively, the data processing system may be configured to read and/or to generate, for each of the subjects, data representing a two-dimensional outline of at least a portion of the respective subject and/or data representing a three-dimensional surface of the respective subject. The data processing system may be further configured to determine, for each of the subjects, the position and/or orientation data depending on the data representing the two-dimensional outline and/or depending on the data representing the three-dimensional surface.

According to a further embodiment, the data processing system is further configured to display, using a graphical user interface, for each of the subjects, a graphical representation. The graphical representation may be determined depending on at least a portion of the determined position and/or orientation parameters of the interior body portion of the respective subject.

The graphical presentation may be displayed on a display device of the data processing system. The graphical representation may be visually indicative of one or more parameters of the position and/or one or more parameters of the orientation of the interior body portion of the respective subject relative to the X-ray sensitive surface.

According to a further embodiment, the graphical representation is a combined graphical representation which comprises a first graphical representation of at least a portion of the interior body portion of the respective subject and a second graphical representation of at least a portion of the X-ray sensitive surface. The first and second graphical representations may be displayed in an overlaid fashion.

According to a further embodiment, the data processing system is further configured to generate, for each of the subjects, positioning data for controlling the X-ray sensitive device for adjusting the relative position and/or the relative orientation between the interior body portion of the respective subject and the X-ray sensitive surface. The positioning data may be generated depending on the determined position and/or orientation parameters of the interior body portion of the respective subject. The data processing system may further be configured to output the positioning data. A detector system may be configured to receive the positioning data and to adapt one or more parameters of a position, a size and/or a shape of the X-ray sensitive surface depending on the position data. Examples for parameters of a size are but are not limited to: a radius of a circle, a magnification and an area. Examples for parameters of a shape are but are not limited to: a ratio of side lengths of a rectangle or an eccentricity of an ellipse.

Embodiments of the present disclosure pertain to an X-ray detection system for a projection X-ray imaging system. The X-ray detection system comprises the system for automatically or interactively adjusting the relative position and/or relative orientation between the subject under inspection and the X-ray sensitive surface according to any one of the preceding embodiments. The X-ray detection system further includes a detector for acquiring, for each of the subjects, an X-ray radiograph of at least a portion of the respective subject under inspection. The X-ray sensitive device is a radiation dose sensor which is provided by the detector or which is provided separately from the detector. The X-ray detection system is configured to control an X-ray exposure of the detector depending on sensor signals generated using the radiation dose sensor.

According to a further embodiment, the controlling of the X-ray exposure comprises generating a deactivation signal for an X-ray source depending on the sensor signals, which are generated using the radiation dose sensor. The deactivation signal may deactivate generation of X-rays by the X-ray source.

According to a further embodiment, the radiation dose sensor is provided by the detector so that the X-ray sensitive surface is provided by one or more X-ray sensitive pixels of the detector. The detector may be configured to measure an X-ray dose received by one or more X-ray sensitive pixels of the detector. The detector may be configured to measure the X-ray dose using a binning of one or more X-ray sensitive pixels of the detector.

According to an embodiment, the radiation dose sensor is provided by the detector so that the X-ray sensitive surface is provided by one or more X-ray sensitive pixels of the detector. The X-ray detection system may be configured to adapt a position, a shape and/or a size of the X-ray sensitive surface. The X-ray detection system is configured to perform, for each of the subjects, the adaptation depending on the one or more of the determined position and/or orientation parameters of the interior body portion of the respective subject. The adaptation may be performed so that the adapted X-ray sensitive surface corresponds to a portion of an X-ray image acquired by the detector. The image portion which corresponds to the X-ray sensitive surface may be at least a part of an image region, which corresponds to the imaged interior body portion.

According to an embodiment, the adaptation of the position, the size and/or the shape comprises selecting the pixels of the detector, which provide the radiation-sensitive surface of the radiation dose sensor. At least a portion of the pixels may be binned together for generating the sensor signals of the radiation dose sensor. The pixels, which provide the radiation-sensitive surface may form or may be composed of a plurality of pixel groups, wherein for each of the pixel groups, the pixels of the respective group may be binned together. Each of the pixel groups may represent a sub-array. Each of the sub-arrays may be a rectangular or square array.

Embodiments of the present disclosure pertain to a method for automatically or interactively adjusting a relative position and/or a relative orientation between an interior body portion of each of a plurality of subjects under inspection and an X-ray sensitive surface of an X-ray sensitive device using a data processing system. The method comprises reading and/or generating, for each of the subjects and using the data processing system, position and/or orientation data which are indicative of one or more parameters of a position and/or an orientation of the respective subject. The method further comprises determining, for each of the subjects and using the data processing system, one or more position and/or orientation parameters of an interior body portion of the respective subject. For each of the subjects, the determining of the one or more position and/or orientation parameters of the interior body portion of the respective subject is performed depending on same model data which are indicative of one or more parameters of an interior position and/or an interior orientation of an interior body portion of a model subject, within a body of the model subject.

The method may further includes generating the model data depending on data generated using computer tomography and/or data generated using planar X-ray radiography.

Embodiments of the present disclosure pertain to a program element for automatically or interactively adjusting a relative position and/or a relative orientation between each of a plurality of subjects under inspection and an X-ray receiving surface of an X-ray sensitive device using a data processing system. The program element, when being executed by a processor of the data processing system, is adapted to carry out: reading and/or generating, for each of the subjects and using the data processing system, position and/or orientation data which are indicative of one or more parameters of a position and/or an orientation of the respective subject relative to the X-ray sensitive surface; determining, for each of the subjects and using the data processing system, one or more position and/or orientation parameters of an interior body portion of the respective subject relative to the X-ray sensitive surface. For each of the subjects, the determining of the one or more position and/or orientation parameters of the interior body portion of the respective subject is performed depending on same model data which are indicative of one or more parameters of an interior position and/or an interior orientation of an interior body portion of a model subject within a body of the model subject.

Embodiments of the present disclosure further pertain to a computer readable medium having stored thereon the computer program element of the preceding embodiments.

### BRIEF DESCRIPTION OF THE DRAWINGS

Fig. 1 is a schematic illustration of an X-ray imaging system for projection radiography, which includes an X-ray detection system according one of the exemplary embodiments described herein;
Figs. 2A and 2B schematically illustrate a portion of an X-ray detection system according to a first exemplary embodiment;
Fig. 3 schematically illustrates the determination of position parameters of an interior body portion performed using the X-ray detection system according to the first exemplary embodiment;
Fig. 4 schematically illustrates model data sets used by the X-ray detection system according to the first exemplary embodiment for determining position parameters of the interior body portion;
Fig. 5 is a schematic illustration of a portion of an X-ray detection system according to a second exemplary embodiment.

### DETAILED DESCRIPTION OF THE EMBODIMENTS

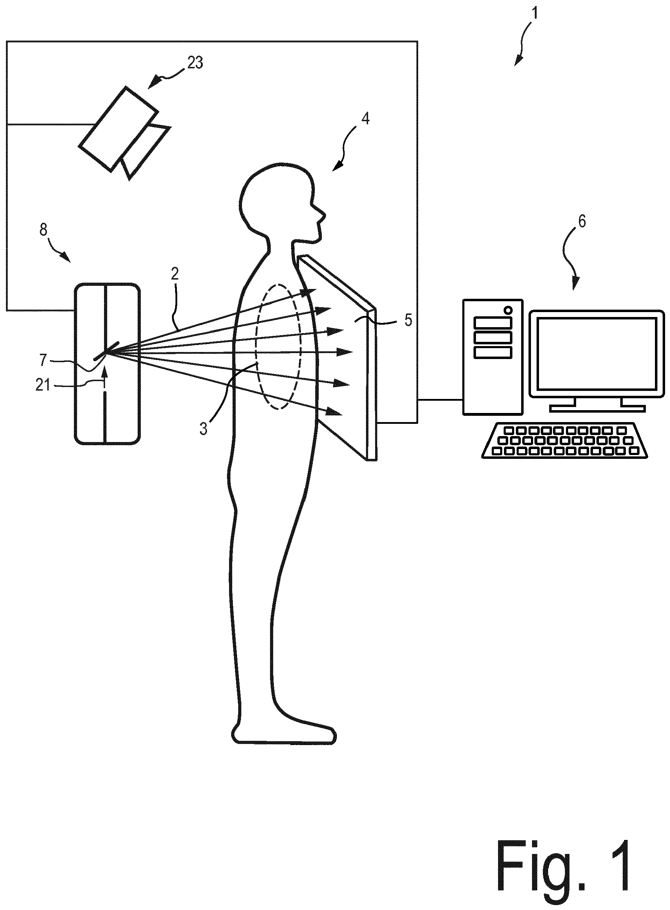

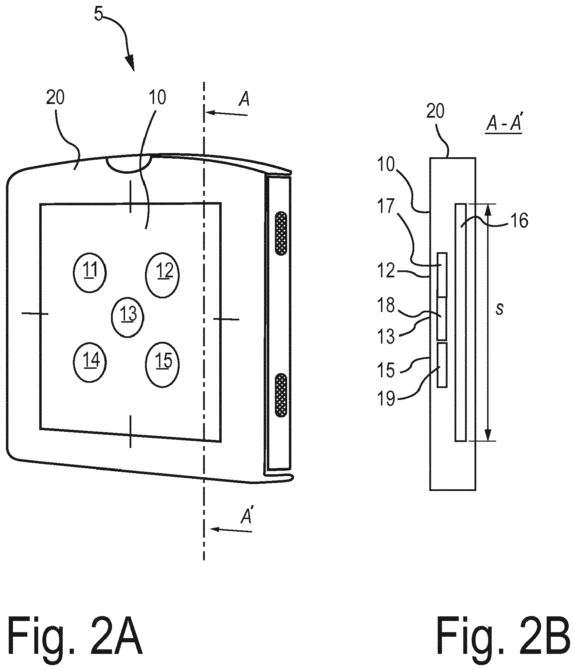

Fig. 1 is a schematic illustration of an X-ray imaging system 1 for projection radiography, which includes an X-ray detection system according to exemplary embodiments described herein. The configurations of X-ray detection systems according to a first and a second exemplary embodiment are discussed in more detail further below in connection with Figs. 2A to 5.

The X-ray imaging system 1 includes an X-ray source 8, which is a tube assembly. The X-ray source 8 emits X-rays 2 toward a subject 4 under inspection so as to irradiate a body portion of the subject 4, such as the subject's lungs 3. The X-ray source 8 emits the X-rays from a small emission region 7 having a diameter of less than 5 millimeters, or less than 2 millimeters onto which an electron beam 21 generated by the X-ray source 8 impinges. The emission region 7 is thereby considerably smaller than an extent of the imaged body portion of the subject 4. The X-ray source 8 is therefore a good approximation of a point radiation source. The body portion to be radiographed (i.e. the lungs 3 in the example, which is shown in Fig. 1) is arranged between the X-ray source 8 and a detector 5, which is configured to generate an image indicative of the intensity distribution of the X-rays incident on the detector 5. The detector 5 may be an analog detector, such as a film, or a digital X-ray detector.

It is conceivable that the aspects and techniques of the present disclosure can be applied to X-ray projection radiography, which images other body portions. Further examples of such body portions are the skull or one or more extremities, such as at least a portion of the upper limbs or at least a portion of the lower limbs.

It is further conceivable that the aspects and techniques of the present disclosure can be applied to other imaging systems such as computer tomography systems.

Figs. 2A and 2B are schematic illustrations of a portion of an X-ray detection system according to a first exemplary embodiment. The X-ray detection system is implemented in the X-ray imaging system 1, which has been discussed with reference to Fig. 1. Fig. 2A is a schematic perspective view of a support structure 20 of the X-ray detection system. Fig. 2B is a schematic cross-sectional view through the support structure 20. The support structure may be a housing or a frame.

As is illustrated in Fig. 2A, the support structure 20 includes a front panel 10 which includes marks 11, 12, 13, 14 and 15, each of which indicating a position, a size and a shape of a radiation-sensitive surface of an X-ray sensitive device. Each of the X-ray sensitive devices is a radiation dose sensor, which is configured to measure an X-ray radiation dose. A controller (not shown) of the detection system is configured to read measurement signals, which are generated by one or more of the radiation dose sensors and to control, depending on the signals, an exposure of an X-ray detector, which is supported by the support structure 20 and which is configured to generate image data representing a radiograph.

The controller may be configured so that controlling the exposure of the X-ray detector includes deactivating an impingement of an electron beam 21 generated by the X-ray source 8 onto a target of the X-ray source, in particular onto the emission region 4 of the target. Additionally or alternatively, controlling the exposure may include controlling a current of the electron beam 21. By way of example, the controller is configured so that controlling the exposure of the X-ray detector includes controlling the X-ray source 8 (shown in Fig. 1) to deactivate an acceleration voltage which accelerates the electron beam 21 toward the emission region 4. Additionally or alternatively, the impingement of the electron beam 21 may be deactivated by controlling a potential of a grid which is disposed in the beam path of the electron beam between the cathode and the emission region 4.

Fig. 2B shows a cross-sectional view through the support structure 20 taken along section line A-A' which is illustrated in Fig. 2A. As can be seen from Fig. 2B, at a position, which is indicated by the marks 11, 12, 13, 14 and 15 and behind the front panel 10, X-ray sensitive devices are supported by the support structure 20, of which the X-ray sensitive devices 17, 18 and 19 are visible in the cross-sectional view of Fig. 2B. The X-ray sensitive devices, such as the devices 17, 18 and 19 are arranged, as seen in a direction of the X-rays, between the X-ray source 1 (shown in Fig. 1) and the X-ray detector 16 (shown in Fig. 2B), which is supported by the support structure 20 and thereby held in a fixed relation relative to the X-ray sensitive devices (such as the devices 17, 18 and 19 shown in Fig. 2B).

In the exemplary embodiment, X-ray detector 16, includes a solid state image sensor. The image sensor may be configured as an array of pixels, which may be controlled by a digital image processor. The array may, for example, have a side length s of at least 10 cm or at least 15 cm. The pixels, may be organized in a regular array of rows and columns. Each of the pixels may have a size which is smaller than 500 micrometers, or smaller than 300 micrometers, or smaller than 200 micrometers. The size of the pixels may be greater than 50 micrometers. Examples for the image sensor are but are not limited to: CCD area image sensors, CMOS area image sensors and a photodiode array or a network of photodiodes.

The pixels may be sensitive to X-rays. Alternatively, it is also conceivable that the detector 16 includes a scintillator, such as a phosphor scintillator, which is arranged, as seen in a direction of the X-rays, between the subject and the image sensor. The pixels of the image sensor, may have a spectral response, which matches at least a portion of the emission band of the scintillator.

However, also other configurations of the detector 16 are conceivable. By way of example, the detector 16 may include a film. The film may be sensitive to X-rays or may be arranged, as seen in a direction of the X-rays, downstream of a scintillator.

In the first exemplary embodiment, each of the radiation dose sensors is configured as an ionization chamber or one or more photodiodes. In the exemplary embodiment, which is illustrated in Fig. 2B, the radiation dose sensors are arranged, as seen in a direction of the X-rays, upstream of the detector 16. However, it is also conceivable that the radiation dose sensors are arranged downstream of the detector 16. By way of example, the radiation dose sensors may detect X-rays which pass through the image detector in regions, which are formed by gaps between adjacent pixels caused by a reduced pixel fill factor. The X-ray detection system of the first exemplary embodiment may further include a Bucky-Potter grid, which is not shown in Figs. 2A and 2B.

It has been shown by the inventor that in conventional X-ray imaging systems, it is difficult to reliably position the subject under inspection relative to the radiation dose sensors so that the radiation dose sensors are located at a desired position relative to an interior body portion of the subject, which is to be imaged. By way of example, for lung radiographs, the preferred position of the radiation dose sensor is within the lung field. For radiographic inspection of bones, the preferred location is, as seen in a direction of the X-rays, downstream of the bones.

On the other hand, the inventor has shown that it is possible to configure the imaging system so that consistent and reproducible automatic control of the X-ray exposure is delivered through efficient positioning of the patient relative to the radiation dose sensors.

As is illustrated in Fig. 1, the imaging system 1 includes a position and/or orientation data acquisition system 23, which is in signal communication with the data processing system 6 and which is configured to acquire measurement data, which are used by the data processing system 6 to determine position and/or orientation data, which are indicative of one or more parameters of a position and/or an orientation of the subject 4 relative to the X-ray sensitive devices of the detector 5.

By way of example, the position and/or orientation data acquisition system 23 includes one or more cameras, which are mounted in a fixed relation relative to the detector 5. If more than one camera is provided, imaging axes of he cameras (in particular their optical axes) may be angled relative to each other. Additionally or alternatively, the position and/or orientation acquisition system 23 may include a laser scanner, which is configured to scan a surface of the subject. Each of the cameras may include an optical system for imaging the subject onto an image sensor of the respective camera.

The position and/or orientation data acquisition system 23 may be configured to generate two-dimensional and/or three-dimensional image data. The data processing system 6 may be configured to analyze the image data to determine the position and/or orientation data. By way of example, the data processing system 6 may be configured to perform a segmentation of the image data.

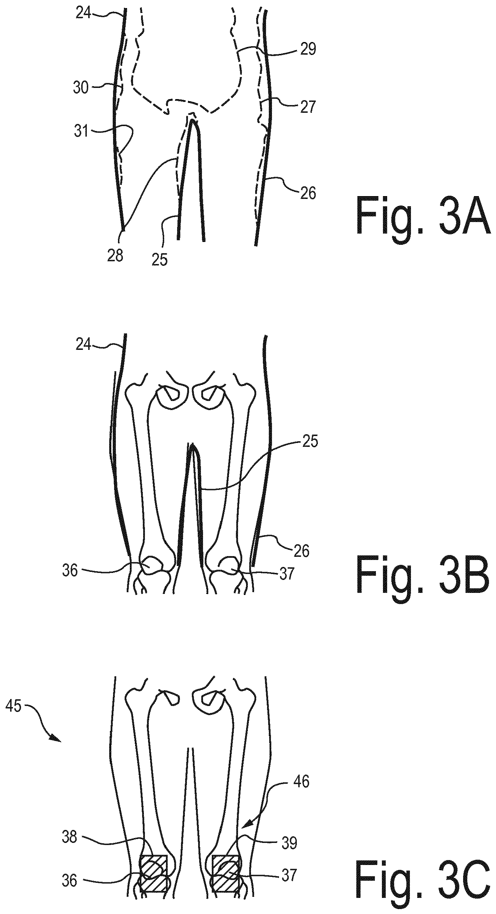

Depending on the data which are generated by the position and/or orientation data acquisition system 23, the data processing system 6 may be configured to determine a two-dimensional outline, such as the solid outline lines 24, 25 and 26 in Fig. 3A. Additionally or alternatively, the data processing system 6 may be configured to determine a three-dimensional surface map of an outer surface of at least a portion of the body of the subject 4, such as is represented in Fig. 3A by the dashed contour lines 27, 28, 29 and 30 (i.e. curves along which a function has a constant value). It is to be noted that the contour map, which is represented by the contour lines 27, 28, 29 and 30, are only one example for a data set which represents a three-dimensional surface.

The two-dimensional outline lines and/or the surface map of the three-dimensional outer surface represent position and/or orientation data which are indicative of one or more parameters of a position and/or orientation of the subject 4.

The data processing system 6 is configured to use the position and/or orientation data for determining one or more position and/or orientation parameters of an interior body portion of the subject relative to the X-ray sensitive surfaces of the radiation dose sensors. Based on the position and/or orientation parameters of the interior body portion, it is possible for the radiographer, radiologist and/or the data processing system 6 to check, whether the subject's position and/or orientation is correct in order to perform an automatic exposure control, which ensures sufficient image quality and which protects the subject 4 from overexposure.

The inventor has shown that it is possible to determine the one or more position and/or orientation parameters of the interior body portion in an efficient manner depending on model data, which are indicative of one or more parameters of an interior position and/or an interior orientation of the interior body portion within a body of a model subject.

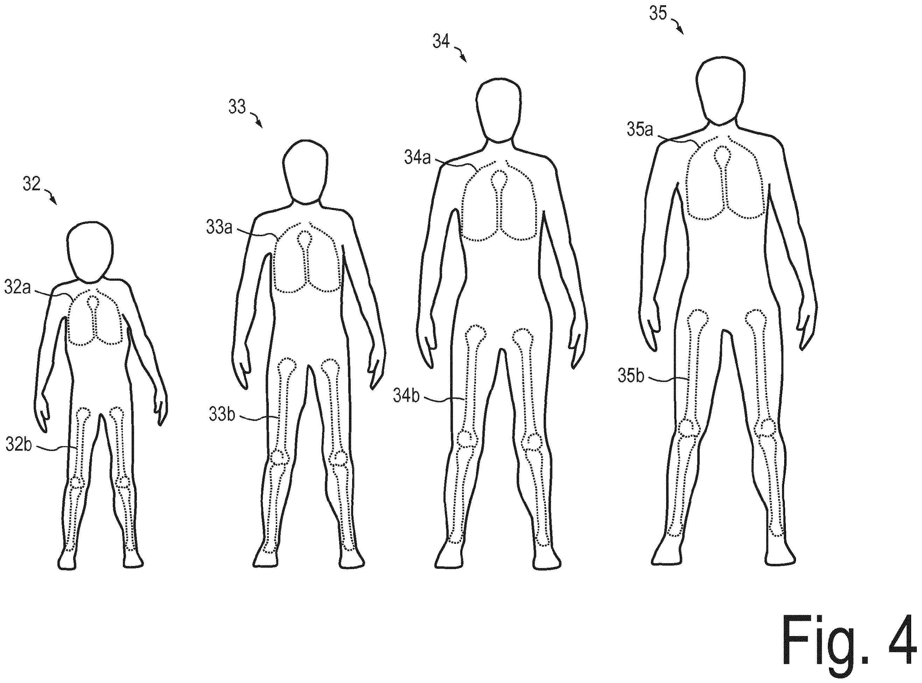

The model data, which are used in the exemplary embodiment, are taken from a database of a plurality of model data sets which is schematically illustrated in Fig. 4. The database may be stored on a storage device of the data processing system 6 and/or may be stored in a remote storage system, which may be accessible by the data processing system 6 via a computer network, such as the Internet and/or a LAN (local area network).

As is schematically illustrated in Fig. 4, the database includes a plurality of model data sets 32, 33, 34 and 35, which relate to different attributes, in particular physical attributes, of model subjects. Examples of such attributes are but are not limited to: gender, age, and body dimensions, such as body height, weight, and body mass index (BMI).

By way of example, as is illustrated in Fig. 4, the data sets 32 and 33 relate to children of different ages and the data sets 34 and 35 relate to adults of different gender. Further data sets may be provided for model subjects, which represent elder persons. Each of the model data sets 32, 33, 34 and 35 is a model for one or more parameters of an interior position and/or an interior orientation of at least one body portion 32a, 32b, 33a, 33b, 34a, 34b, 35a and 35b. By way of example, the model data sets may be acquired using projection radiography and/or computer tomography data. The model data sets may represent an average of data acquired from a plurality of different subjects which relate to a same set of attributes.

In each of the model data sets 32, 33, 34 and 35, the parameters of the interior position and/or interior orientation of the interior body portions 32a, 32b, 33a, 33b, 34a, 34b, 35a and 35b may be measured relative to an outer surface of the model subject. By way of example, the model data of the data sets may be generated using computer tomography data and/or X-ray radiography data. The model data may be generated using data acquired from one or more other subjects than the subject, which is under inspection.

The data processing system is configured to select one or more model data sets 32, 33, 34 and 35 depending on selection data. The data processing system may further be configured to interpolate between two or more selected model data sets. The selection data may be indicative of one or more parameters of attributes of the subject under inspection. Examples for selection data are but are not limited to: gender, age, and body dimensions, such as body height, weight, and body mass index (BMI). Further examples of the selection data are a thickness of a portion of the subject which is illuminated by the X-rays (such as a thickness of the thorax), as seen in a direction of at least a portion of the X-rays. The direction may be measured perpendicular to an image plane of the detector. A further example is thickness of the portion of the subject which is illuminated by the X-rays (such as the thorax), measured in a direction perpendicular to the direction of the portion of the X-rays. The direction perpendicular to the direction of the portion of the X-rays may be measured perpendicular or along a longitudinal axis of the subject.

The data processing system may be configured to receive the selection data from the user via the user interface. Additionally or alternatively, the X-ray imaging system 1 may be configured to measure one or more parameters of attributes of the subject 4 under inspection. By way of example, the X-ray imaging system includes one or more sensors (not shown in Fig. 1), which measure the height of the body of the subject 4.

It is also conceivable, that at least a portion of the position and/or orientation data which is acquired by the position and/or orientation data acquisition system 23 (shown in Fig. 1) is used by the data processing system 6 for generating the selection data.

As is schematically illustrated in Fig. 3B, based on the model data, it is possible to derive one or more parameters of a relative position and/or orientation between the the subject on the one hand (represented by the outline lines 24, 25 and 27) and the interior body portion to be radiographed, on the other hand, such as the knee joint 28, 29 shown in Fig. 3B, or the lungs 3 (as is shown in Fig. 1).

By way of example, the data processing system 6 may be configured to perform a statistical fit between the model data on the one hand and the position and/or orientation data of the subject on the other hand to determine one or more position and/or orientation parameters of the interior body portion of the subject. It is further conceivable that the model data are configured to be adaptable to a posture of the body of the subject. By way of example, the model data may include one or more variation parameters which can be varied during the statistical fitting process.

The relative position may be a position in a plane, which is parallel to an image plane of the detector. However, it is also conceivable that the position parameters may include a relative position in a direction perpendicular to the image plane.

As is illustrated in Fig. 3C, thereby, it is possible to determine the positions of the knee joints 38, 39 relative to the positions of X-ray sensitive surfaces represented by the graphical representations 38, 39.

The data processing system is further configured to display, on a display device of the data processing system, a graphical representation 45, which is indicative of one or more parameters of a position and/or an orientation of the internal body portion relative to one or more X-ray sensitive surfaces of the radiation dose sensors. This allows the radiographer or radiologist to adjust the subject's position and/or orientation so that an optimal radiation dose measurement is ensured.

By way of example, as is illustrated in Fig. 3C, the graphical representation is a combined graphical representation 46 of at least one body portion and one or more graphical representations of at least one X-ray sensitive surface (such as the graphical representations 38 and 39) in an overlaid fashion. The graphical representations 38 and 39 of the X-ray sensitive surfaces show the position, the shape and/or the size of the X-ray sensitive surfaces.

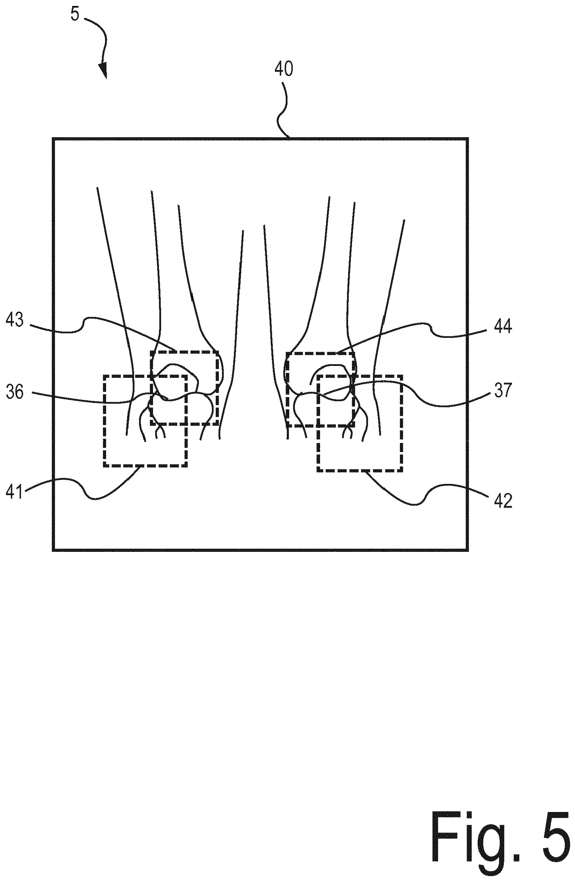

Fig. 5 is a schematic illustration of a detector 5 of a detector system according to a second exemplary embodiment. The detector system of the second exemplary embodiment may be implemented in an X-ray imaging system, as has been described in connection with the first embodiment and which is illustrated in Fig. 1.

The detector 5 of the second exemplary embodiment includes a solid state image sensor 40. The solid-state image sensor 40 may be sensitive to light, which is emitted from a scintillator (not shown in Fig. 5) and which is arranged, as seen in a direction of the X-rays, upstream of the image sensor 40. Alternatively, it is conceivable, that the image sensor 40 is configured as a solid state direct X-ray image sensor, which is sensitive to X-rays so that it is not necessary to provide a scintillator for converting the X-rays into photons of a different wavelength.

The circuitry provided on the image sensor 40 is configured to generate a dose sensing signal which is generated by binning a plurality of pixels, which are arranged in a sub-array, wherein the read-out process of the dose sensing signal does not destroy the image read-out signal of the individual pixels. The circuitry thereby forms an array of superpixels, each of which being a sub-array. By way of example, each sub-array which forms a superpixel may be a rectangular or square array which includes at least 9 pixels or at least 100 pixels or at least 1,000 pixels. The pixels of the image sensor 40 may be grouped into super pixels so that the super pixels may include all pixels of the image sensor 40. However, it is also conceivable that only a portion of the pixels of the image sensor 40 form part of a superpixel.

The image sensor 40 is further configured so that for each of the superpixels, a sampling rate for reading out the dose sensing signal of the respective superpixel is faster than 500 Hz, faster than one 1 kHz, or faster than 5 kHz. This allows a fast and efficient read-out process for the dose sensing signal so that efficient exposure control can be provided.

An arrangement of such an image sensor is disclosed, for example in US patent no. 7,601,961 to Franklin et al., the contents of which is incorporated herein by reference.

The image sensor 40 is in signal communication with the data processing system (designated with reference numeral 6 in the first embodiment shown in Fig. 1). The data processing system is configured to select a number of super pixels to be used for controlling the exposure during an imaging process.

The data processing system is configured to select, depending on the determined position and/or orientation parameters of the interior body portion (e.g. the positions of the knee joints in a plane parallel to the image plane of the detector) one or more superpixels, which are to be used in the imaging process for controlling the exposure.

By way of example, as can be seen from the example illustrated in Fig. 5, the data processing system selects two rectangular arrays of superpixels 43 and 44, which are used for controlling the exposure when imaging two knee joints 36 and 37 of the subject.

Depending on the anatomical dimensions of the subject, the position and orientation of the subject, and further depending on the body portion to be radiographed, it is possible to adjust the position, shape and/or size of the area represented by the arrays of superpixels 43 and 44, which are used for controlling the exposure. This is schematically illustrated by the array of superpixels 41 and 42 which are used for controlling the exposure in a radiograph of a different subject which has different body dimensions and a different position and/or orientation relative to the detector during image acquisition.

It has been shown by the inventor that this allows more accurate dose sensing measurements, which are adapted to the anatomical features of the subject under inspection as well as to the posture of the subject during imaging. Specifically, it has been shown by the inventor that this overcomes a major drawback in conventional techniques of automatic exposure control. The large and fixed position of conventional radiation dose sensors make automatic exposure control unsuitable for pediatric imaging. Notably, the constant growth that occurs during childhood results in changing body proportions so that fixed radiation dose sensors cannot effectively be used for all age ranges. The X-ray detection system according to the second exemplary embodiment, however, makes it possible to adapt the X-ray sensitive surfaces of the radiation dose sensors so that efficient exposure control is provided for a wide variety of anatomical variances. The adaptation may be performed so that the adapted X-ray sensitive surface corresponds to a portion of an X-ray image acquired by the detector. The image portion which corresponds to the X-ray sensitive surface may be at least a part of an image region, which corresponds to the interior body portion for which the position and/or orientation parameters has been determined.

It is to be noted that it is not necessary that the circuitry of the image sensor 40 provides superpixels for performing the radiation dose measurements. It is conceivable that image sensors are used which allow a non-destructive readout of one or more individual pixels during image acquisition. It is further conceivable that the pixels or superpixels which are used for controlling the exposure are not a rectangular or square array but represent other shapes on the active surface of the image sensor. Specifically, the data processing system may be configured to adapt the position, shape and/or size of the X-ray sensitive surfaces provided by the pixels used for the radiation dose measurement to the position, shape and/or size of the interior body portion to be radiographed. Further, the pixels used for the radiation dose measurement need not to be organized in pixel clusters but may be isolated from each other with other pixels of the image sensor being arranged between them.

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from the protective scope of the claims of the present invention. In the claims, the word "comprising" does not exclude other elements or steps, and the indefinite article "a" or "an" does not exclude a plurality. Any reference signs in the claims should not be construed as limiting the scope.

## Claims

1. A system for automatically or interactively adjusting, for each of a plurality of subjects (4), a relative position and/or a relative orientation between an interior body portion of the respective subject (4) under inspection and an X-ray sensitive surface of an X-ray sensitive device (17, 18, 19);
wherein the system comprises a data processing system (6) which is configured to:
read and/or determine, for each of the subjects (4), position and/or orientation data which are indicative of one or more parameters of a position and/or an orientation of the respective subject (4);
determine, for each of the subjects (4), one or more position and/or orientation parameters of an interior body portion (3, 36, 37) of the respective subject (4);
wherein the data processing system (6) is configured to determine, for each of the subjects (4), the one or more position and/or orientation parameters of the interior body portion (3) of the respective subject (4) further depending on same model data;
wherein the model data are indicative of one or more parameters of an interior position and/or an interior orientation of an interior body portion (3, 36, 37) of a model subject within a body of the model subject.

2. The system of claim 1, wherein the data processing system (6) is configured to:
read and/or generate, for each of the subjects (4), selection data for selecting the model data from a plurality of model data sets (32, 33, 34, 35), each of which being associated with one or more model subjects; and to
select, for each of the subjects (4), the model data depending on the selection data wherein the selection data are indicative of, or are determined by the data processing system depending on, one or parameters of attributes of the respective subject (4) under inspection.

3. The system of claim 1 or 2,
wherein the X-ray sensitive device (17, 18, 19) is a radiation dose sensor which is configured to generate radiation dose measurement signals which are indicative of a radiation dose absorbed by the X-ray sensitive surface; and
wherein the system further includes a controller which is configured to receive the radiation dose signals and to generate a control signal for controlling an exposure of an X-ray source depending on the radiation dose measurement signals.

4. The system of any one of the preceding claims, wherein the data processing system (6) is configured to read and/or generate, for each of the subjects (4), two-dimensional and/or three-dimensional image data which show at least a portion of a body of the respective subject (4) and to determine, for each of the subjects (4), the position and/or orientation data depending on the image data, acquired for the respective subject (4).

5. The system of any one of the preceding claims,
wherein the data processing system (6) is further configured to display, using a graphical user interface, for each of the subjects (4) a graphical representation (45); and
wherein the graphical representation is determined depending on at least a portion of the determined position and/or orientation parameters of the interior body portion (3, 36, 37) of the respective subject (4).

6. The system of claim 5, wherein the graphical representation (45) is a combined graphical representation which comprises a graphical representation (46) of at least a portion of the interior body portion of the respective subject (4) and a graphical representation of at least a portion of the X-ray sensitive surface (38, 39).

7. The system of any one of the preceding claims, wherein the data processing system (6) is further configured to:
generate, for each of the subjects (4), positioning data for controlling the X-ray sensitive device for adjusting the relative position and/or the relative orientation between the interior body portion of the respective subject (4) and the X-ray sensitive surface;
wherein the positioning data are generated depending on the determined position and/or orientation parameters of the interior body portion of the respective subject (4); and to
output the positioning data.

8. An X-ray detection system for a projection X-ray imaging system, wherein the X-ray detection system comprises:
the system for automatically or interactively adjusting the relative position and/or relative orientation between the subjects (4) under inspection and the X-ray sensitive surface according to any one of the preceding claims;
a detector (5) for acquiring, for each of the subjects (4) an X-ray radiograph of at least a portion of the respective subject (4);
wherein the X-ray sensitive device is a radiation dose sensor which is provided by the detector (5) or which is provided separately from the detector (5);
wherein the X-ray detection system is configured to control an X-ray exposure of the detector (5) depending on sensor signals generated using the radiation dose sensor.

9. The X-ray detection system of claim 8, wherein the controlling of the X-ray exposure comprises generating a deactivation signal for an X-ray source depending on the sensor signals, which are generated using the radiation dose sensor.

10. The X-ray detection system according to claim 8 or 9,
wherein the radiation dose sensor is provided by the detector (5) so that the X-ray sensitive surface is provided by one or more X-ray sensitive pixels of the detector (5);
wherein the detector (5) is configured to measure the X-ray dose using a binning of one or more X-ray sensitive pixels of the detector (5).

11. The X-ray detection system according to any one of claims 8 to 10,
wherein the radiation dose sensor is provided by the detector (5) so that the X-ray sensitive surface is provided by one or more X-ray sensitive pixels of the detector (5);
wherein the X-ray detection system is configured to adapt a position, a size and/or a shape of the X-ray sensitive surface; and
wherein the X-ray detection system is configured to perform, for each of the subjects (4) the adaptation depending on the one or more of the determined position and/or orientation parameters of the interior body portion of the respective subject (4).

12. The X-ray detector system according to claim 11, wherein the adaptation of the position, the size and/or the shape comprises selecting the pixels of the detector (5), which provide the radiation-sensitive surface of the radiation dose sensor.

13. A method for automatically or interactively adjusting a relative position and/or a relative orientation between an interior body portion of each of a plurality of subjects (4) under inspection and an X-ray sensitive surface of an X-ray sensitive device (17, 18, 19) using a data processing system (6);
wherein the method comprises:
reading and/or generating, for each of subjects (4) and using the data processing system (6), position and/or orientation data which are indicative of one or more parameters of a position and/or an orientation of the respective subject (4);
determining, for each of subjects (4) and using the data processing system (6), one or more position and/or orientation parameters of an interior body portion of the respective subject (4);
wherein for each of the subjects (4), the determining of the one or more position and/or orientation parameters of the interior body portion of the respective subject (4) is performed depending on same model data;
wherein the model data are indicative of one or more parameters of an interior position and/or an interior orientation of the interior body portion of a model subject within a body of the model subject.

14. A program element for automatically or interactively adjusting a relative position and/or a relative orientation between an interior body portion of each of a plurality of subjects (4) under inspection and an X-ray receiving surface of an X-ray sensitive device (17, 18, 19) using a data processing system (6);
wherein the program element, when being executed by a processor of the data processing system (6), is adapted to carry out:
reading and/or generating, for each of the subjects (4) and using the data processing system (6), position and/or orientation data which are indicative of one or more parameters of a position and/or an orientation of the subject;
determining, for each of the subjects (4) and using the data processing system (6), one or more position and/or orientation parameters of an interior body portion (3, 36, 37) of the respective subject;
wherein for each of the subjects (4), the determining of the one or more position and/or orientation parameters of the interior body portion (3, 36, 37) of the respective subject (4) is performed depending on same model data;
wherein the model data are indicative of one or more parameters of an interior position and/or an interior orientation of the interior body portion (3, 36, 37) of a model subject within a body of the model subject.

15. A computer readable medium having stored thereon the computer program element of claim 14.
